(19) Europäisches Patentamt
European Patent Office
Office européen des brevets

(11) Veröffentlichungsnummer: **0 402 796 A2**

(12) # EUROPÄISCHE PATENTANMELDUNG

(21) Anmeldenummer: **90110919.9**

(51) Int. Cl.[5]: **G01N 33/543**

(22) Anmeldetag: **09.06.90**

(30) Priorität: **14.06.89 DE 3919393**

(43) Veröffentlichungstag der Anmeldung:
**19.12.90 Patentblatt 90/51**

(84) Benannte Vertragsstaaten:
**AT BE CH DE DK ES FR GB GR IT LI LU NL SE**

(71) Anmelder: **HOECHST AKTIENGESELLSCHAFT**
**Postfach 80 03 20**
**D-6230 Frankfurt am Main 80(DE)**

(72) Erfinder: **Hachmann, Henning, Dr.**
**Merzinger Weg 1a**
**D-6000 frankfurt am Main(DE)**
Erfinder: **Molz, Peter, Dr.**
**Kaiser-Wilhelm-Ring 44**
**D-6500 Mainz(DE)**
Erfinder: **Neuenhofer, Stephan, Dr.**
**Falkensteiner Weg 16**
**D-6231 Sulzbach(DE)**
Erfinder: **Schnorr, Gerd, Dr.**
**Auf dem Lattigkopf 23**
**D-6368 Bad Vilbel(DE)**
Erfinder: **Simons, Guido, Dr.**
**Talstrasse 24**
**D-6507 Ingelheim(DE)**
Erfinder: **Skrzipczyk, Heinz-Jürgen, Dr.**
**Am Schellberg 16**
**D-6232 Bad Soden am Taunus(DE)**
Erfinder: **Weimer, Kurt E., Dr.**
**Hundertmorgenring 156**
**D-6082 Mörfelden(DE)**

(54) **Verfahren zur Stabilisierung von auf Festphasen immobilisierten biologisch aktiven Substanzen.**

(57) Die Erfindung betrifft ein Verfahren zur Stabilisierung von biologisch aktiven Substanzen, die auf einem Träger immobilisiert sind, wobei die Festphase mit der immobilisierten biologisch aktiven Substanz zur Stabilisierung mit einer Lösung in Kontakt gebracht wird, die Polyanetholsulfonsäure und/oder deren Salze enthält. Insbesondere handelt es sich bei den biologisch aktiven Substanzen um Antikörper.

RIA-gnost CEA 1-Schritt

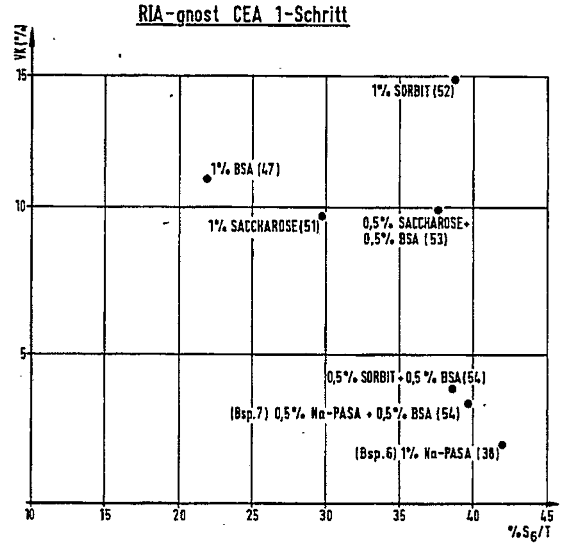


BELASTUNG: 6 TAGE KÜHLRAUM
6 TAGE KLIMARAUM
6 TAGE TROPENRAUM

DIE WERTE IN KLAMMERN ENTSPRECHEN $\%S_6/T$ VOR BELASTUNG

EP 0 402 796 A2

## Verfahren zur Stabilisierung von auf Festphasen immobilisierten biologisch aktiven Substanzen

Die vorliegende Erfindung betrifft ein Verfahren zur Stabilisierung von biologisch aktiven Substanzen, die auf einem Träger (Festphase) immobilisiert sind, sowie Träger zur Durchführung immunchemischer Nachweisverfahren, auf denen die mit dem erfindungsgemäßen Verfahren stabilisierten biologisch aktiven Substanzen immobilisiert sind.

Zahlreiche immunchemische Nachweisverfahren zur quantitativen und qualitativen Bestimmung von therapeutisch und/oder diagnostisch wichtigen Substanzen basieren auf dem Festphasen-Prinzip, wobei ein wasserunlöslicher Träger (Festphase) mit biologisch aktivem Material beschichtet wird, d.h. das biologisch aktive Material wird auf dem Träger immobilisiert. Dadurch ist es sehr einfach möglich, den gebundenen von dem ungebundenen Anteil eines Analyten abzutrennen, sowie den gebundenen Analyten von allen störenden Faktoren der Probe zu trennen. Als Festphasen kommen z.B. synthetische oder natürliche Polymere wie Polystyrol, Polypropylen, PVC oder Latex in verschiedenen geometrischen Ausführungsformen, wie Röhrchen, Kugeln oder Mikrotitrationsplatten in Betracht.

Die beschichteten Träger weisen jedoch den Nachteil auf, daß bei mehr oder weniger langen Lagerzeiten die Aktivität der biologisch aktiven Substanzen nachläßt. Diese Aktivitätsverluste können produktionsseitig nur bis zu einem bestimmten Grenzwert durch erhöhten Einsatz von Beschichtungsmaterial ausgeglichen werden. Zur Vermeidung dieses Nachteils kann das biologisch aktive Material stabilisiert (vergütet) werden.

Verschiedene Verfahren zur Stabilisierung des biologisch aktiven Materials sind beschrieben, so in der DE-B 29 10 707 die Verwendung von Zucker, in der EP-A 0 140 489 die Verwendung von Zucker und Proteinen, in EP-A 0 170 983 die Verwendung von hydrolisiertem Ovalbumin, in der EP-A 0 133 976 die Verwendung von Zuckersäuren und deren Salzen. Die erreichten Stabilitäten sind jedoch in vielerlei Hinsicht noch nicht befriedigend, was sich in einer Abnahme der Bindungskapazität zu dem nachzuweisenden Analyten - ausgedrückt als Quotient der Bindung eines beliebigen Standards und der Totalaktivität-und in einer nicht mehr gewährleisteten Reproduzierbarkeit der Messungen - ausgedrückt als Variationskoeffizient $V_k$ - bemerkbar macht.

Die der Erfindung zugrundeliegende Aufgabe bestand darin, ein Stabilisierungsmaterial zu finden, das eine verbesserte Stabilisierung des immobilisierten biologisch aktiven Materials sowie eine gute Reproduzierbarkeit der Messungen gewährleistet. Insbesondere soll die Reproduzierbarkeit der Messungen auch nach längerer oder unsachgemäßer Lagerung der beschichteten Festphase gewährleistet sein.

Überraschenderweise ergab die Vergütung mit Polyanetholsulfonsäure alleine oder mit anderen Stabilisierungsmaterialien zusammen eine hohe Stabilität des immobilisierten biologisch aktiven Materials und eine ausgezeichnete Reproduzierbarkeit der Messungen.

Die vorliegende Erfindung betrifft demnach ein Verfahren zur Stabilisierung von biologisch aktiven Substanzen, die auf einem Träger immobilisiert sind, das dadurch gekennzeichnet ist, daß die Festphase mit der immobilisierten biologisch aktiven Substanz zur Stabilisierung mit einer Lösung in Kontakt gebracht wird, die Polyanetholsulfonsäure und/oder deren Salze enthält.

Polyanetholsulfonsäure (PASA) ist ein Polymerisat der Anetholsulfansäure der Formel I

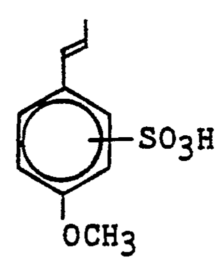 (I)

mit einem Molekulargewicht von ca. 8000 bis 12000. Für den erfindungsgemäßen Einsatz von PASA empfiehlt sich solches mit einem Molekulargewicht von 9000 bis 11000.

Erfindungsgemäß können neben der freien Polyanetholsulfonsäure auch deren Salze eingesetzt werden. Als Salze der Polyanetholsulfonsäure kommen bevorzugt die Alkalimetallsalze und hier insbesondere die Natrium- und/oder Kaliumsalze zur Anwendung.

Als wasserunlösliche Träger (Festphase) sind beispielsweise geeignet: natilrliche und synthetische, organische und anorganische Polymere, wie Polystyrol, Polypropylen, Polyvinylchlorid, Polyvinylidenfluorid,

Latex, Polyacrylamid, Magnetit oder poröses Glaspulver. Die Träger können z. B. als Teströhrchen, Titrierplatte, Küvette, Stab oder Kugel ausgebildet sein. Besonders geeignete Träger für die biologisch aktiven Substanzen sind Polystyrolröhrchen und Polystyrolkugeln oder Latexkugeln. Sofern zweckdienlich, können die Träger auch aus magnetischem bzw. magnetisierbarem Material bestehen oder dieses zumindest enthalten, was den Vorteil hat, daß diese Träger leicht mittels eines Magneten von anderen Systemen abgetrennt werden können.

Als immunometrische Bestimmungsverfahren kommen Radioimmunoassays (RIA), Enzymimmunoassays (EIA) oder Chemiluminezenzimmunoassays (CIA oder LIA) in Betracht.

Das biologisch aktive Material, mit dem der Träger beschichtet ist, ist beispielsweise ein Antigen, ein Antikörper oder ein Hapten. Bevorzugte Antikörper sind solche gegen carcinoembryonales Antigen (CEA). Insbesondere bevorzugt sind monoklonale Antikörper gegen CEA, die in an sich bekannter Weise hergestellt werden.

Zur Stabilisierung der auf dem Träger immobilisierten biologisch aktiven Substanz geht man am besten so vor, daß man den nach bekannten Verfahren mit biologisch aktiven Substanzen beschichteten Träger mit einer Lösung in Kontakt bringt, die Polyanetholsulfonsäure und/oder eines ihrer Salze und gegebenenfalls noch andere Stabilisierungsmittel (Vergütungsmittel) enthält. Als weitere Vergütungsmittel, die neben PASA und/oder deren Salze eingesetzt werden können, seien genannt: Polymethylhydrogensiloxan, Polyethylenglycol (insbesondere solches mit einem Molekulargewicht von 20.000 - 50.000), Polyacrylsäure, Polyvinylpyrrolidon, Polyvinylpolypyrrolidon, Polyvinylalkohol, Polypropylenglycol, Lecithin, Sorbit, Tylose, Saccharose, Casein, Gelatine, Rinderserumalbumin. Der pH-Wert der Vergütungslösung sollte zwischen 5 und 9 liegen, insbesondere zwischen 7 und 8. Zur Erreichung dieses pH-Wertes wird PASA und/oder eines oder mehrere ihrer Salze und gegebenenfalls das oder die anderen Vergütungsmittel in einer Pufferlösung gelöst, welche auf den gewünschten pH-Wert eingestellt ist. Als Puffer kommen beispielsweise Tris-(hydroxymethyl)-aminomethan, Citronensäure, tri-Natriumcitrat, Natriumazid, Salzsäure oder Natronlauge, oder auch beliebige Gemische dieser Substanzen in Betracht. Die gebrauchsfertige gepufferte Vergütungslösung enthält PASA und/oder eines oder mehrere ihrer Salze in einer Konzentration von 0.01 bis 50g/l (bezogen auf die Vergütungslösung), bevorzugt von 0,1 bis 20 g/l, besonders bevorzugt von 1 bis 10 g/l. Enthält die gebrauchsfertige gepufferte Vergütungslösung auch noch weitere Vergütungsmittel, so liegen diese in Konzentrationen von 0,1 bis 40 g/l, bevorzugt von 1 bis 20 g/l, besonders bevorzugt von 5 bis 15 g/l (bezogen auf die Vergütungslösung) vor.

Die Vergütung findet durch Kontaktieren des beschichteten Trägers mit der Vergütungslösung statt. Je nach geometrischer Ausgestaltung der beschichteten Träger werden diese entweder insgesamt von der Vergütungslösung bedeckt (z. B. wenn es sich um Kugeln, Stäbchen etc. handelt) oder die Vergütungslösung wird in die Träger hineingegeben (z.B. wenn es sich um Röhrchen, Küvetten etc., die von innen beschichtet sind, handelt). Die Einwirkzeit beträgt 5-50 Stunden, bevorzugt 10-30 Stunden, besonders bevorzugt 15-25 Stunden. Die Vergütung erfolgt bei Temperaturen von 18-28° C, bevorzugt von 20-26° C. Anschließend wird die Vergütungslösung entfernt und die beschichteten vergüteten Träger getrocknet, bevorzugt durch Vakuumtrocknung, beispielsweise 1-5 Stunden bei Raumtemperatur und einem Druck von 5-20 mbar. Die Lagerung der erfindungsgemäß vergilteten Träger kann bei Raumtemperatur erfolgen. Gegenüber herkömmlich vergilteten Trägern, beispielsweise solchen, die mit Rinderserumalbumin, Saccharose oder Sorbit vergüteten wurden, weisen die erfindungsgemäß vergüteten Träger eine verbesserte Stabilität und eine hervorragende Reproduzierbarkeit der Messungen auf.

## Beispiele

### Beispiel 1

Herstellung des Vergütungspuffers:

6 g Tris-(hydroxymethyl)aminomethan, 3,2 g Citronensäure, 10 g tri-Natriumcitrat • $2H_2O$ und 1 g Natriumazid werden in einem Liter entionisiertem Wasser gelöst und der pH-Wert mit HCl bzw. NaOH auf 7,5 eingestellt.

### Beispiel 2

Herstellung der Vergütungslösung:

Die gewünschte Menge des Natriumsalzes der Polyanetholsulfonsäure Na-PASA (Sigma, Sigma Chemie, Deisenhofen, oder FLUKA, Feinchemikalien GmbH, Neu-Ulm) wird in dem Vergütungspuffer gelöst. Lösungen mit folgenden Na- PASA- Gehalten wurden hergestellt:

| Beispiel-Nr. | Gew.-% Na-PASA |
|---|---|
| 2a | 0,1 |
| 2b | 0,2 |
| 2c | 0,5 |
| 2d | 1,0 |

Ferner wurde eine Vergütungslösung, die 0, 5% Na- PASA und 0,5% Rinderserumalbumin (BSA) enthielt, hergestellt (Beispiel 2e).

Beispiel 3

Polystyrolröhrchen (Länge 75 mm, Durchmesser 12 mm) werden auf der Innenseite ca. 5 mm hoch (ca. 200 µl) in herkömmlicher Weise mit monoklonalen Mausantikörpern gegen CEA beschichtet. In diese Röhrchen werden 300 µl der Vergütungslösung aus Beispiel 2a gegeben und diese abgedeckt 20 Stunden bei Raumtemperatur stehen gelassen. anschließend wird die Vergütungslösung abgesaugt und die Röhrchen werden 3 Stunden bei Raumtemperatur und einem Druck von 10 mbar getrocknet.

Beispiel 4

Analog zu Beispiel 3 werden beschichtete Polystyrolröhrchen mit der Vergütungslösung aus Beispiel 2b vergütet.

Beispiel 5

Analog zu Beispiel 3 werden beschichtete Polystyrolröhrchen mit der Vergütungslösung aus Beispiel 2c vergütet.

Beispiel 6

Analog zu Beispiel 3 werden beschichtete Polystyrolröhrchen mit der Vergütungslösung aus Beispiel 2d vergütet.

Beispiel 7

Analog zu Beispiel 3 werden beschichtete Polystyrolröhrchen mit der Vergütungslösung aus Beispiel 2e vergütet.

**Bestimmung der Stabilität und der Reproduzierbarkeit**

Die vergüteten Röhrchen aus den Beispielen 3 bis 7 wurden einer Temperaturbelastung unterworfen. Dazu wurden sie Jeweils 6 Tage in einem Kühlraum (4 °C, 60% Luftfeuchtigkeit), anschließend 6 Tage in einem Tropenraum (40 °C, 80% Luftfeuchtigkeit) und daraufhin noch 6 Tage in einem Klimaraum (alternierend: 12 h, 40 °C, 80% Luftfeuchtigkeit; 12 h, 5 °C, 60% Luftfeuchtigkeit) gelagert. Anschließend wurden von allen vergüteten Röhrchen aus den Beispielen 3 bis 7 die Bindungswerte ($S_6/T$ in %) und der

Variationskoeffizient (VK in %) gemessen. Der Bindungswert $S_6/T$ ergibt sich aus dem Quotienten von: Signal des gebundenen Tracers bei Verwendung des höchsten Standards ($S_6$ vom RIA-gnost CEA) und Signal der pro Röhrchen eingesetzten Tracermenge (Totalaktivität T), und der Variationskoeffizient VK (in %) errechnet sich aus der Standardabweichung einer 10-fach Messung (SD) und dem Mittelwert dieser 10-fach Messung (Mean) zu (SD/Mean)•100. Die erhaltenen Ergebnisse sind in Tabelle 1 (Belastung: Klimaraum, und gegebenenfalls Tropenraum) und Figur 1 dargestellt. Als Vergleich sind in der Figur 1 die Ergebnisse von herkömmlich vergüteten Röhrchen-hergestellt wie in Beispiel 3 beschrieben, unter Verwendung von Saccharose, Sorbit, Rinderserumalbumin und Gemischen dieser Verbindungen - mit aufgeführt. Tabelle 1 enthält als Vergleichsbeispiel ein mit 1% BSA vergiltetes Röhrchen.

Die erfindungsgemäß vergilteten Röhrchen weisen im Vergleich zu den herkömmlich vergüteten Röhrchen eine bessere Stabilität und somit eine bessere Reproduzierbarkeit der Messungen auf.

Tabelle 1

| Röhrchenstabilität nach Belastung (RIA-gnost CEA 1-Schritt) | | | | | |
|---|---|---|---|---|---|
| Vergütet mit | Anfangs-Bindung ($S_6/T$) | Lagerung im | | $S_6/T$ nach Belastung (10fach Best.) | |
| | | Klimaraum | Tropenraum | % Bindung ($S_6/T$) | VK% |
| 1,0 % BSA (vgl. Bsp.) | 52 % | 19 d | | 15 | 16,9 |
| 0,1 % Na-PASA (Bsp.3) | 46 % | 19 d | | 46 | 2,1 |
| 0,2 % Na-PASA (Bsp.4) | 46 % | 19 d | | 45 | 1,7 |
| 0,5 % Na-PASA (Bsp.5) | 47 % | 19 d | | 45 | 2,3 |
| 1,0 % Na-PASA (Bsp.6) | 47 % | 19 d | | 41 | 1,7 |
| 1,0 % BSA (vgl. Bsp.) | 47 % | 6d | 6 d | 22 | 11,0 |
| 1,0 % Na-PASA (Bsp.6) | 38 % | 6d | 6 d | 42 | 2,0 |

**Ansprüche**

1. Verfahren zur Stabilisierung von biologisch aktiven Substanzen, die auf einem Träger immobilisiert sind, dadurch gekennzeichnet, daß die Festphase mit der immobilisierten biologisch aktiven Substanz zur Stabilisierung mit einer Lösung in Kontakt gebracht wird, die Polyanetholsulfonsäure und/oder deren Salze enthält.

2. Verfahren gemäß Anspruch 1, dadurch gekennzeichnet, daß die Lösung noch weitere Vergütungsmittel enthält.

3. Verfahren nach Anspruch 1 oder 2, dadurch gekennzeichnet, daß die Lösung einen Puffer enthält.

4. Verfahren nach einem oder mehreren der Ansprüche 1 bis 3, dadurch gekennzeichnet, daß die Festphase anschließend getrocknet wird.

5. Verfahren nach einem oder mehreren der Ansprüche 1 bis U, dadurch gekennzeichnet, daß die biologisch aktive Substanz ein Antikörper ist.

6. Verfahren nach einem oder mehreren der Ansprüche 1 bis U, dadurch gekennzeichnet, daß die biologisch aktive Substanz ein monoklonaler Antikörper ist.

7. Verfahren nach einem oder mehreren der Ansprüche 1 bis U, dadurch gekennzeichnet, daß die biologisch aktive Substanz ein Antigen ist.

8. Verfahren nach einem oder mehreren der Ansprüche 1 bis 7, dadurch gekennzeichnet, daß die Festphase aus Latexpartikeln besteht oder ein Polystyrolröhrchen ist.

9. Verfahren nach einem oder mehreren der Ansprüche 1 bis 7, dadurch gekennzeichnet, daß die Festphase magnetisch anziehbar ist.

10. Verfahren nach einem oder mehreren der Ansprüche 1 bis 7, dadurch gekennzeichnet, daß die Festphase ein synthetisches Polymer ist.

11. Verfahren zur Herstellung eines Trägers zur Durchführung immunchemischer Nachweisverfahren, wobei eine biologisch aktive Substanz auf dem Träger immobilisiert wird, dadurch gekennzeichnet, daß die immobilisierte biologisch aktive Substanz gemäß einem Verfahren nach einem oder mehreren der Ansprüche 1 bis 10 stabilisiert wird.

12. Träger zur Durchführung immunchemischer Nachweisverfahren, erhältlich nach Anspruch 11.

13. Verwendung eines Trägers nach Anspruch 11 in einem Radioimmunoassay, Enzymimmunoassay oder Chemiluminessenzimmunoassay.

Patentansprüche für folgende Vertragsstaaten: ES, GR

1. Verfahren zur Stabilisierung von biologisch aktiven Substanzen, die auf einem Träger immobilisiert sind, dadurch gekennzeichnet, daß die Festphase mit der immobilisierten biologisch aktiven Substanz zur Stabilisierung mit einer Lösung in Kontakt gebracht wird, die Polyanetholsulfonsäure und/oder deren Salze enthält.

2. Verfahren gemäß Anspruch 1, dadurch gekennzeichnet, daß die Lösung noch weitere Vergütungsmittel enthält.

3. Verfahren nach Anspruch 1 oder 2, dadurch gekennzeichnet, daß die Lösung einen Puffer enthält.

4. Verfahren nach einem oder mehreren der Ansprüche 1 bis 3, dadurch gekennzeichnet, daß die Festphase anschließend getrocknet wird.

5. Verfahren nach einem oder mehreren der Ansprüche 1 bis 4, dadurch gekennzeichnet, daß die biologisch aktive Substanz ein Antikörper ist.

6. Verfahren nach einem oder mehreren der Ansprüche 1 bis 4, dadurch gekennzeichnet, daß die biologisch aktive Substanz ein monoklonaler Antikörper ist.

7. Verfahren nach einem oder mehreren der Ansprüche 1 bis 4, dadurch gekennzeichnet, daß die biologisch aktive Substanz ein Antigen ist.

8. Verfahren nach einem oder mehreren der Ansprüche 1 bis 7, dadurch gekennzeichnet, daß die Festphase aus Latexpartikeln besteht oder ein Polystyrolröhrchen ist.

9. Verfahren nach einem oder mehreren der Ansprüche 1 bis 7, dadurch gekennzeichnet, daß die Festphase magnetisch anziehbar ist.

10. Verfahren nach einem oder mehreren der Ansprüche 1 bis 7, dadurch gekennzeichnet, daß die Festphase ein synthetisches Polymer ist.

11. Verfahren zur Herstellung eines Trägers zur Durchführung immunchemischer Nachweisverfahren, wobei eine biologisch aktive Substanz auf dem Träger immobilisiert wird, dadurch gekennzeichnet, daß die immobilisierte biologisch aktive Substanz gemäß einem Verfahren nach einem oder mehreren der Ansprüche 1 bis 10 stabilisiert wird.

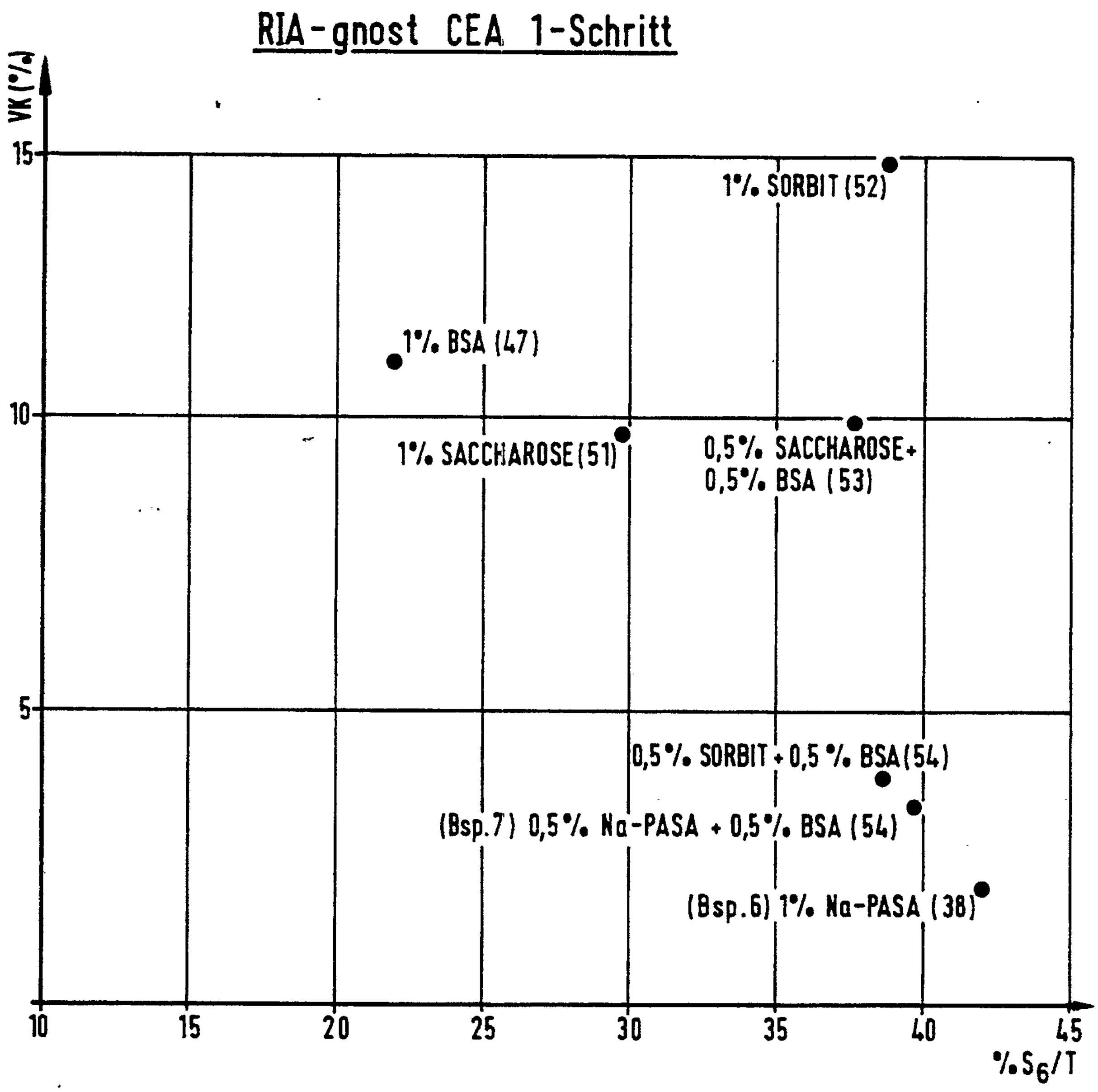


BELASTUNG: 6 TAGE KÜHLRAUM
6 TAGE KLIMARAUM
6 TAGE TROPENRAUM

DIE WERTE IN KLAMMERN ENTSPRECHEN %$S_6$/T <u>VOR</u> BELASTUNG